# EUROPEAN PATENT APPLICATION

(11) **EP 3 238 774 A1**
(43) Date of publication of application: **01.11.2017**
(21) Application number: 17162315.0
(22) Date of filing: 22.03.2017
(51) Int. Cl.: A61N 1/04, A61N 1/36

(54) **PROCESS FOR ESTABLISHING AN ELECTROSTIMULATION PROTOCOL FOR HEADACHE CONTROL, AND THE RESPECTIVE PORTABLE ELECTROSTIMULATION EQUIPMENT FOR HEADACHE CONTROL USING SAID PROTOCOL**

(30) Priority: 31.03.2016 BR 102016007242
(71) Applicant: Medecell S.A., Montevideo (UY)
(72) Inventor: Marques de Oliveira, Mauricio, São Paulo (BR); Bighetti, Moacyr Ramos, São Paulo (BR)
(74) Representative: Michalski Hüttermann & Partner Patentanwälte mbB

(57) **Abstract**

Said process and electrostimulation equipment being more specifically employed in the control of headaches resulting from migraine and of secondary pain to temporomandibular joint disorders, said process predicting the development of an electrostimulation protocol in which the variation of electric pulse variation is made in a random manner, respecting the limits of stimulation efficacy, to reduce the physiological phenomenon of nerve fiber accommodation upon stimuli; a portable electrostimulation equipment, formed by an electrostimulation set (1) and a support (2) is provided, the former consisting of a cylindrical compartment (3) housing an electric circuit board (4) and a disposable coin-shaped lithium-ion battery (5), model CR20XX, in whose back face there are recesses (6a), which house the respective electrodes (6), suitably covered by the respective gel layers protected by removable protective sheets (7); the electrostimulation set (1) being engaged with the medial longitudinal groove (14) of the support (2), which configures the rail along which aid set is moved; the following internal components are also provided: Power source module (15), step-up regulator module (16), micro controller module (17), power supply seal module (18), boost source module (19), H-bridge module (20), and electrode output module (21).

## Description

### TECHNICAL FIELD

As known in the state of the art, Transcutaneous Electrical Nerve Stimulation (TENS) is a well-known and already consecrated modality of non-medicament and non-invasive treatment for pain control in several etiologies. Such treatment consists in the placement of electrodes in determined regions of the human body, and in the application of electric pulses with the purpose of stimulating the nerve fibers (or nerves); this electrical stimulation produces an analgesic effect generating a reduction or even total elimination of the pain.

This treatment mode has already been used in several clinical scenarios for the treatment of several acute and chronic pain conditions, and have been well accepted among health professionals. Among the many indications of TENS, there are headaches of different etiologies. It is an alternative or an adjuvant to the medicated analgesic treatment, also reducing the need for anti-inflammatories.

### DESCRIPTION OF THE PRIOR ART

Currently, the treatment of different types of headaches is mainly performed through the use of medicaments (SCOTTISH INTERCOLLEGIATE, 2008). Only in the last decades, scientific works about the use of electrostimulation in headache treatment and prevention were carried out (ALLAIS, 2003; SCOTTISH INTERCOLLEGIATE, 2008).

In the literature, we have described several sites of application of electrostimulation for the control of headache of different etiologies. The stimulation of the occipital nerve is an alternative for the control of primary headaches refractory to drug treatments (LAMBRU&MATHARU, 2012; BARTSCH, PAEMELEIRE & GOADSBY, 2009). Migraines can be treated and prevented by supra-orbital stimulation (SCHOENEN, 2013; SILVA et al., 2012). The use of electrostimulation is well characterized for pain resulting from temporomandibular joint (TMA) (GROSSMANN, 2012, KATO et a I., 2006).

The usual electrostimulation equipment can be divided into two large groups: the bench equipment, powered by the electric network, and the portable equipment, powered by batteries.

For the specific purposes of treating headaches and similar conditions, self-applicable portable equipment has been used, which, upon professional advice, can be used by the user himself/herself, at home or at work.

Among the already known portable electrostimulation devices specifically designed to control headaches, let us mention the ones provided in documents N°s. BR PI 0517186-5 (corresponding to WO 2006/063417), WO 2006/051370, US 8.428.734 e US 8.702.584; in these documents, the electrostimulation device itself, formed by the electrodes responsible for the electrostimulation, by the electronic circuit that generates the electric pulses and by the energy-generating source (battery), is mounted on a support applied to the user's head.

The portable electrostimulation equipment currently existing in the market (including the ones mounted on head supports such as those provided for in the aforementioned documents) have a major drawback: the sizes of the electrostimulation set are still large due to the sizes of the batteries used. In fact, the operations carried out by the electrical stimulation equipment require a large current consumption, which is the reason why the batteries used therein need to have sufficient capacity to meet this demand, and to power the equipment for a period that is considered acceptable. The batteries that meet these requirements show considerable sizes. For this reason, the use of smaller, disposable batteries such as coin-shaped lithium-ion batteries has never been feasible, since these, having lower charge capacity, would substantially reduce the shelf life of the equipment.

Another disadvantage of the batteries commonly used in these portable equipment is the need to recharge them. In fact, it happens quite often situations in which the equipment is discharged when pain affects the user; in these cases, the user does not always have a place to recharge said device, and often, depending on the intensity of the pain, he/she cannot wait for this operation, and ends up resorting more readily to medicines. Ideally, the user should have an electrostimulation equipment, which needs no charging, and is ready for use anytime and anywhere.

On the other hand, it is also known that to be effective in the treatment of pain, electrical stimuli (or pulses) must meet a series of requirements in terms of their intensity (or amplitude) [in volts (V)], their frequency [in hertz (Hz)], their width (or duration) [in microseconds (µs)], its waveform, and the used stimulation module.

In the scientific literature, there are several reports showing that analgesia induced by electrical pulses occurs within a very elastic range of the previously mentioned parameters.

In fact, the numerous electrostimulation devices known in the art usually apply electrical pulses whose parameters are found in the following ranges:
- current intensity: 1-50 mA (charged at 500Ω));
- frequency: 1-250 Hz;
- pulse-width (or duration): 10-1000 µs;
- waveform: single-phase, symmetrical biphasic, asymmetrical biphasic; and - stimulation mode: continuous or intermittent.

Although there is extensive literature attesting to the efficacy of TENS, the mechanism of action is not fully understood, and the Theory of the Pain Portal and the Central Release of Endorphins are the most accepted mechanisms of action by the scientific community.

A well-known physiological phenomenon is the one of the nerve fiber accommodation at the electric stimulus. It is the refractoriness of the nerve cell membrane when the stimulus is applied in the same phase and with fixed parameters of intensity, frequency, and pulse width. In this case, the stimulation ceases to be effective and the analgesic effect does not occur.

To avoid the nerve fiber accommodation, several strategies have been developed, among them:
- inversion of the polarity of the electrical pulses;
- waveform change;
- variation of the frequency or intensity (amplitude) value of the electrical pulses.

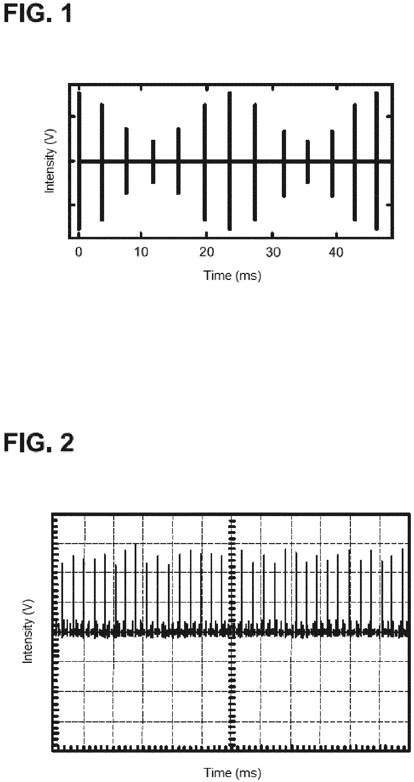

When the variation of the intensity (amplitude) of the electrical pulses is used as a strategy to prevent the accommodation of the nerve fibers, this variation is always done in a regular way in time (as shown in the graphical representation of attached Figure 1)

However, even when adopting this measure, there is still some degree of nerve fiber accommodation, precisely because there is a regular temporal repetition of said intensity variations. As a function of the plasticity and adaptive capacity of the cell membranes of the nerve fibers, regular intervals of variation allow said membranes to adapt, also causing the nerve fibers to be accommodated, thereby losing or reducing the analgesic effect.

This is another inconvenient of the electrostimulation equipment currently available in the market: the difficulty in avoiding nerve fiber accommodation when applying the electrical pulses.

Therefore, it would be desirable to develop some kind of protocol for the application of electrical stimuli that would be able to reduce nerve fiber accommodation in a more efficient way, and, therefore, to guarantee the prolonged analgesic effect.

On the other hand, it would also be desirable to obtain a portable equipment for electrostimulation application that consumes less current when applying electrical pulses, allowing the use of batteries of smaller size and capacity, resulting in a disposable cheaper and smaller equipment, guaranteeing better portability characteristics than the prior art equipment known up to now.

### PURPOSES OF THE INVENTION

In order to achieve these purposes, an innovative electrostimulation protocol was developed, in which the intensity variation of the applied electrical pulses was randomly performed, respecting the limits stimulation efficacy. With this new protocol, it has been possible to efficiently reduce the accommodation of cell membranes of nerve fibers, increasing electrostimulation efficacy and, thus, the analgesic effect.

Concurrently, this random variation of electrical pulse intensity has substantially reduced current consumption in the operations of the electrostimulation equipment. This feature allowed the use of disposable coin-shaped lithium-ion batteries, model CR20-XX, which have smaller sizes, lower cost and lower charge capacity, but sufficient to meet the current lower current consumption, thanks to the intensity variations determined by the innovative electrostimulation protocol. Thereby, the disposability of the product has become feasible, since it is not necessary to replace the batteries.

Only this family of batteries combines the characteristics of reduced size, sufficient voltage and charge, low environmental impact and reduced cost, which makes it possible to dispose of the equipment.

Thus, since it is now possible to use such disposable batteries, it has been possible to substantially reduce the sizes of the electrostimulation set integrating the equipment which were larger before because they used to depend on batteries with greater capacity, which until then were necessary to provide the electrostimulation of the apparatus. Moreover, with the use of disposable batteries, it has become possible to significantly reduce the cost of the equipment.

On the other hand, the inventor(s) further developed a novel support for said electrostimulation set, said support having an arrangement providing a perfect adjustment to the user's head anatomy, as well as an effective contact of the electrostimulation electrodes with his/her skin, which is usually very oily in several facial zones.

Furthermore, the support was designed to provide the sliding of the electrostimulation set along a rail, to allow the user to change the positioning of the electrostimulation and, thus, the electrodes, providing the possibility of applying stimuli in different anatomical regions, favoring the application in the supraorbital and temporal regions of the head.

### DESCRIPTION OF THE DRAWINGS

To complement the present description, to better understand the characteristics of the subject matter of the patent, a set of drawings accompanies this specification, in which, in an exemplified and nonlimiting manner, the following has been represented:
- Figure 1 is a graphical representation showing the usual strategy for avoiding the nerve fiber accommodation used in the known electrostimulation protocols, that is, the use of intensity (amplitude) variation of the electrical pulses, variation that, to this day, is done regularly over time;
- Figure 2 is another graphical representation, now illustrating the novel strategy to avoid nerve fiber accommodation, provided by this innovative electrostimulation protocol, that is, the use of a random variation of electrical pulse intensity (amplitude), during the application of pulse bursts;
- Figure 3 shows, also by means of a graphical representation, one of the embodiment of this innovative electrostimulation protocol, where pulse bursts with a determined duration and with sequentially inverted polarity are continuously applied;
- Figure 4 illustrates, similarly by means of a graphical representation, other embodiments of this innovative electrostimulation protocol, that is, the intermittent mode, according to which pulse bursts are applied with a determined duration, and with sequentially inverted polarity, although providing a time interval between said pulse bursts, with a determined duration.
- Figure 5 is another graphical representation showing the steps of this innovative electrostimulation protocol;
- Figures 6 and 7 show, from two perspectives, this innovative portable electrostimulation equipment, which includes an electrostimulation set itself, and a head support where said electrostimulation set is installed;
- Figures 8 and 9 show two further perspectives of the equipment, now showing its component in exploded view;
- Figure 10 is a side view of said equipment;
- Figure 11 is a top view of said equipment;
- Figure 12 is an enlarged detail of the equipment, referred to as "Det. A" in the previous figure, showing only the electrostimulation set;
- Figure 13 is a sectional view of said electrostimulation set, referred to by line I-I in Figure 12, which shows the internal components of the electrostimulation set;
- Figure 14 is a block diagram of said equipment, showing its internal electrical components;
- Figure 15 is the electrical scheme of this innovative equipment;
- finally, Figure 16 is a flowchart of the software, specifically developed for this innovative electrostimulation protocol.

### DETAILED DESCRIPTION OF THE INVENTION

This patent of invention relates to a "PROCESS FOR ESTABLISHING AN ELECTROSTIMULATION PROTOCOL FOR HEADACHE CONTROL, AND THE RESPECTIVE PORTABLE ELECTROSTIMULATION EQUIPMENT FOR HEADACHE CONTROL USING SAID PROTOCOL", said electrostimulation process and equipment being more specifically used to control headache resulting from migraine and secondary pain to temporomandibular joint disorders.

Initially, with references to the "PROCESS FOR ESTABILISHING AN ELECTROSTIMULATION PROTOCOL", said process provides the development of an electrostimulation protocol in which intensity variation of the electrical pulses is performed in a random manner, respecting the lower limit of stimulation efficacy, to reduce the physiological phenomenon of nervous fiber accommodation upon receiving stimuli.

More specifically, according to the present process, the electrical pulses have a square wave shape, monopolar polarity, width (or duration) between 60 µs and 100 µs, preferably 80 µs, and a frequency between 40 Hz and 70 Hz, preferably 55 Hz, and Intensity varying randomly within a range of 10 V, preferably 5 V, as shown in attached Figure 2.

Furthermore, the present process discloses two embodiments for this innovative electrostimulation protocol, namely, continuous mode and intermittent mode.

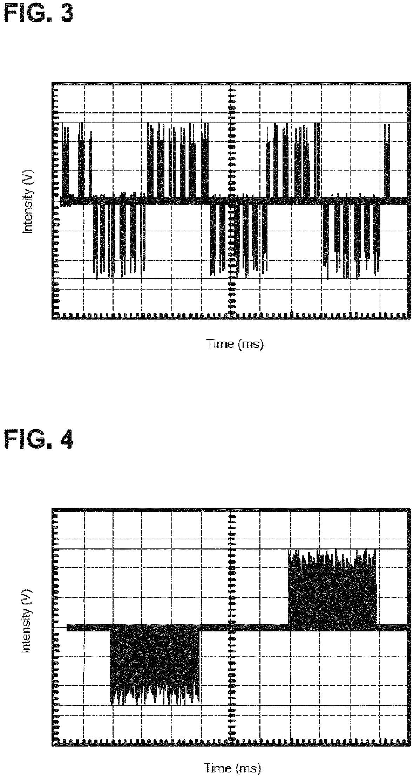

In the continuous mode, shown in the graphical representation of Figure 3, burst trains lasting 500 milliseconds to 2 seconds, preferably 1 second, and with sequentially inverted polarity are continuously applied.

In the intermittent mode, shown in the graphical representation of Figure 4, pulse bursts lasting from 1 to 4 seconds, preferably 3 seconds, and with sequentially inverted polarity are applied, however there is a time interval from 1 to 4 seconds, preferably 3 seconds, between the pulse bursts.

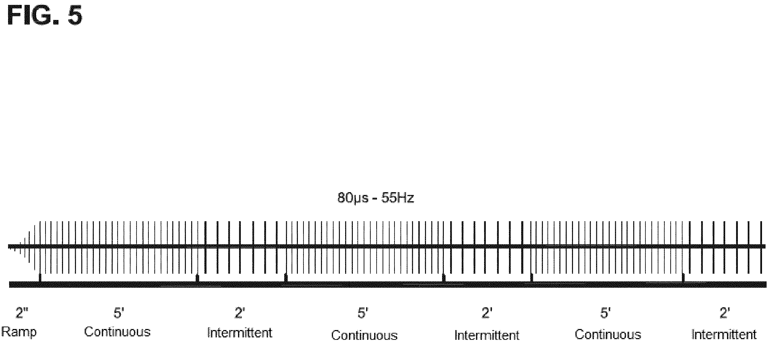

Due to this random variation of electrical pulse intensity throughout the stimulation cycle, the novel electrostimulation protocol for the control of headache comprises the following steps, as shown in attached Figure 5:
a) intensity ramp from 0V to 25V peak (with 500Ω charge), for 2 seconds;
b) 1^{st} continuous mode period, maintained for 5 minutes;
c) 1^{st} intermittent mode period, maintained for 2 minutes;
d) 2^{nd} continuous mode period, maintained for 5 minutes;
e) 2^{nd} intermittent mode period, maintained for 2 minutes;
f) 3^{rd} continuous mode period, maintained for 5 minutes;
g) 3^{rd} intermittent mode period, maintained for 2 minutes;
h) end of the protocol, after 21 minutes and 2 seconds.

In optional embodiments, this innovative protocol discloses the simultaneous use of other strategies used together with the random intensity pulse variation, as a complementary way to further avoid nerve fiber accommodation, and to make current consumption even smaller.

Among the strategies simultaneously employed with the aleatory (or random) variation of the pulse intensity, let us cite the following ones:
- use of monopolar pulse bursts, which allow saving battery charge;
- polarity inversion of the pulse bursts, avoiding muscular fiber accommodation effect;
- inclusion of intermittent modes of interleaved stimulation for the maintenance of analgesia, once the desired effect has been achieved.

By means of this new protocol, in which the intensity of the electric pulses has begun to vary in a random way, it was possible to efficiently reduce nerve fiber accommodation, increasing the effectiveness of the electrostimulation and, thus, of the analgesic effect.

Concurrently, this random variation of electric pulse intensity has further allowed a substantial reduction in current consumption in the operations of the electrostimulation equipment, allowing the use of standard coin-shaped lithium ion batteries, model CR20XX, which are smaller, cheaper and of lower load capacity, but sufficient to meet the current lower current consumption due to random intensity variations, incorporated by this innovative electric stimulation protocol, without the need to replace them.

Thus, it is now possible to use such common ion-lithium batteries, substantially reducing the sizes of the electrostimulation set integrating the equipment, which were greater because of the need of higher capacity batteries to provide the electrostimulation operation of the apparatus.

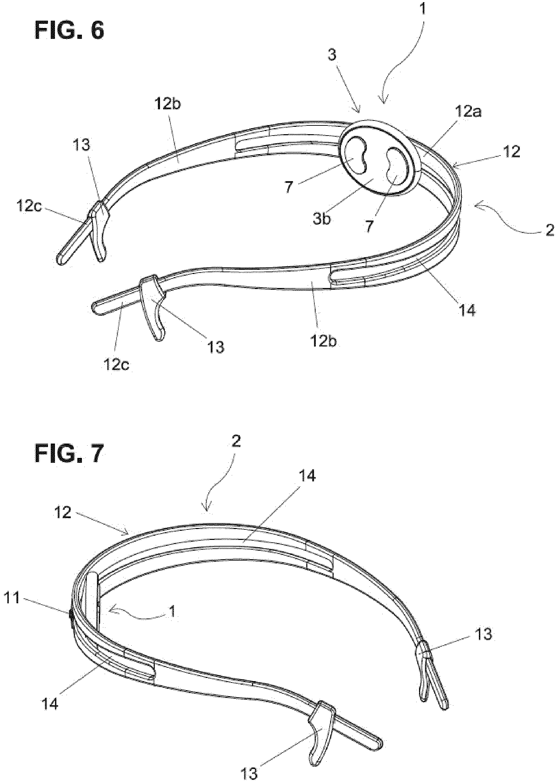

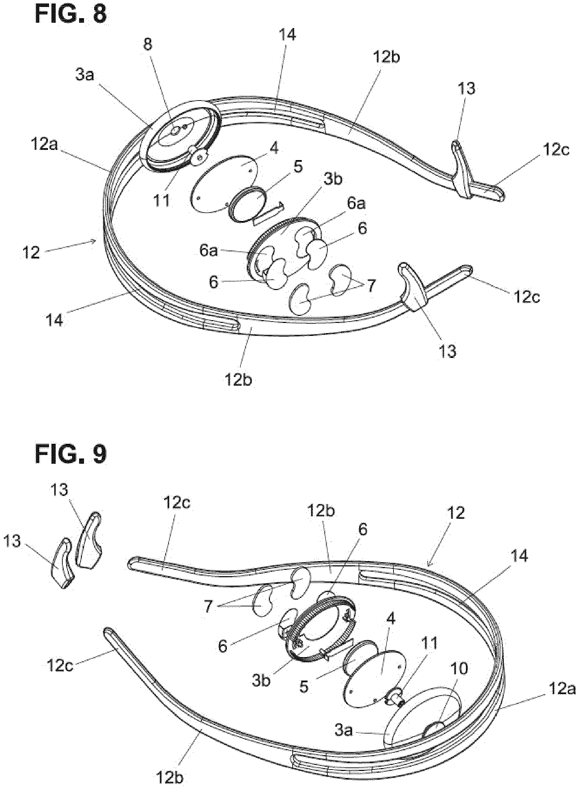

Regarding this "PORTABLE ELECTROSTIMULATION EQUIPMENT FOR HEADACHE CONTROL USING SAID PROTOCOL", said equipment has the details shown in attached Figures 6-12.

Such equipment comprises two parts, an electrostimulation set (1) itself, and a support (2) in which said electrostimulation set (1) is installed, said support (2) configuring the element through which the electrostimulation set (1) is properly positioned and maintained in contact with the regions of the user's head, more specifically with the supraorbital and temporal regions of the user's head.

The electro-stimulator set (1) consists of a cylindrical compartment (3), formed by a front body (3a) and a rear cover (3b), which are engageable with each other, said compartment (3) housing the electronic module of the apparatus, which consists of an electrical circuit board (4) with the components and electrical circuit of the equipment; said compartment (3) also houses the power supply battery (5), which, thanks to the innovative electrostimulation protocol described before, could now be a disposable coin-shaped lithium-ion battery, model CR20XX.

On the outer back face of the cover (3b) of the compartment (3), there are two recesses (6a), which house the respective electrodes (6), suitably covered by the respective gel layers protected by removable protective sheets (7).

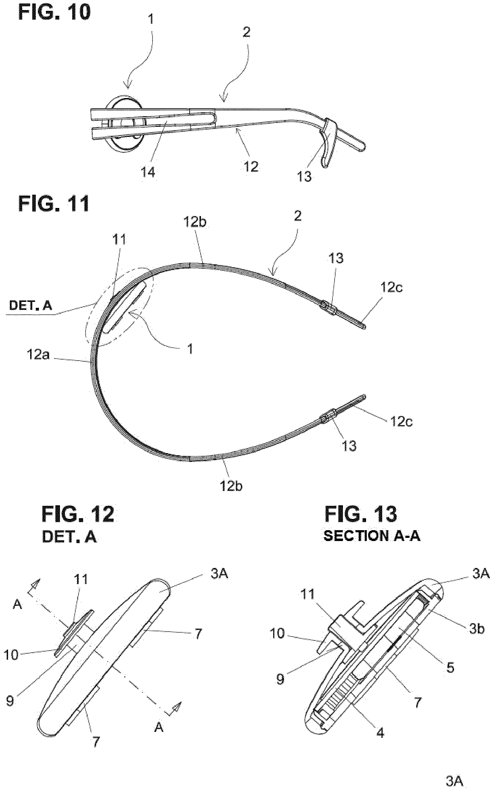

The body (3a) comprising said compartment (3) has a central hole (8), from which a short front small tube (9) protrudes outwardly, whose free end extends outwardly on an external flange (10), said small tube (9) being pierced by a pin (11) constituting the power button of the equipment (see more specifically the enlarged detail and the section of Figures 12 and 13).

Said pin (11) is electrically interconnected to the electric circuit board (4), which is internal to the compartment (3), constituting a touch actuating device whose pressing initiates a sequence of automated electrostimulations, following this innovative electrostimulation protocol, previously described, after which the equipment is automatically switched off.

In turn, the small tube (9) and the flange (10) constitute the means through which the electrostimulation set (1) is fitted to the other piece that integrates this innovative equipment, namely the support (2), described below.

Said support (2) is configured by a lying U-shaped rod (12) whose central portion (12a) -coincident with the user's forehead- becomes arched, when the user places it on his/her head, while its side portions (12b), flanking the temples, extend rearwardly into end portions (12c), which rest on the user's ears.

Properly engaged in the free ends of said end portions (12c) of the rod (12), there are two stylized C-shaped pieces (13), which constitute means for adjusting the support (2) to the head of each user, since said pieces (13) are slidable along said portions (12c) of the rod (12), from back to front, until their arched face anatomically touches the back of the user's ears, ensuring the maintenance of the support on the head, and causing the electrodes (7) to be firmly pressed against the user's skin.

Said rod (12) is further provided with a medial longitudinal groove (14) extending through the entire central portion (12a) and through the side portions (12b) thereof. Said groove (14) configures a rail in which the electrostimulation set (1) is engaged, and along which said set (1) can be moved, as explained below.

To engage the electrostimulation set (1) to the support (2), it is sufficient for the user to force the passage of the flange (10) and the small tube (9) of the former through the medial longitudinal groove (14) of the latter, from the inside out, wherein said flange (10) is disposed on the outer side of the rod (12), while the compartment (3) is disposed on the inner side thereof, with the electrodes (7) facing the user's forehead.

Once this is done, the user makes the electrostimulation set (1) slide along the groove (14), until it reaches a position chosen by him/her or determined by a medical care professional.

Once the position of the electrostimulation set (1) is selected, the user removes the protective sheets (8) which protected the gel layer from the electrodes (7), places the support (2) on his/her head, and adjusts the position of the back pieces (13) by moving them from backwards to forwards along the end portions (12c) of the rod (12), until they lie against the back of their ears; The equipment is thus securely fastened to the head, and the electrodes (6) are firmly pressed against the user's skin.

Thus, to initiate the electrostimulation session, the user presses the power button (11), which starts the sequence of automated stimulation, following this innovative stimulation protocol, previously described, after which the equipment is automatically switched off.

Since the electrostimulation set (1) is slidable along the groove (14) of the support (2), the user can change the position thereof, and then initiate a new electrostimulation session by pressing again the power button (11), which allows the application of electrical pulses in other regions of the user's face, alternating them more particularly between the supraorbital and temporal regions of his/her head.

Due to the substantially reduced sizes of the battery (5) which could now be used in the equipment in question (a coin-shaped lithium-ion rechargeable battery, model CR20XX), as well as to its substantially lower cost, electrostimulation (1) is fully disposable. Hence, said electrostimulation set (1) is used during the duration of the battery charge (5), after which it is removed from the support (2) and replaced by another set (1), which is easily engaged with the support (2), as already described above.

As mentioned earlier, when the power button (11) is pressed, the above-described electrostimulation protocol is started again, automatically switching off the device at the end of said protocol.

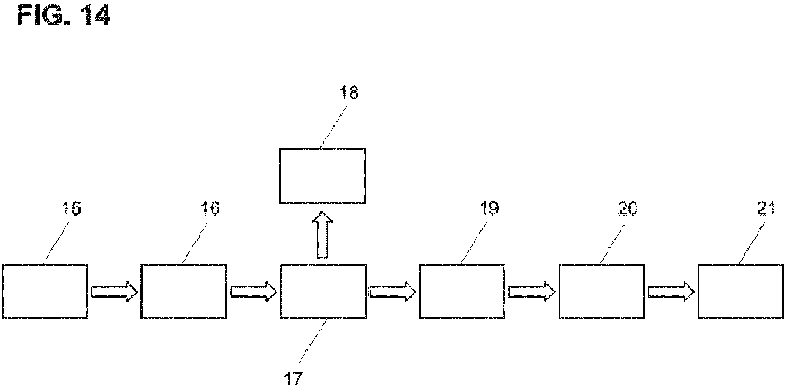

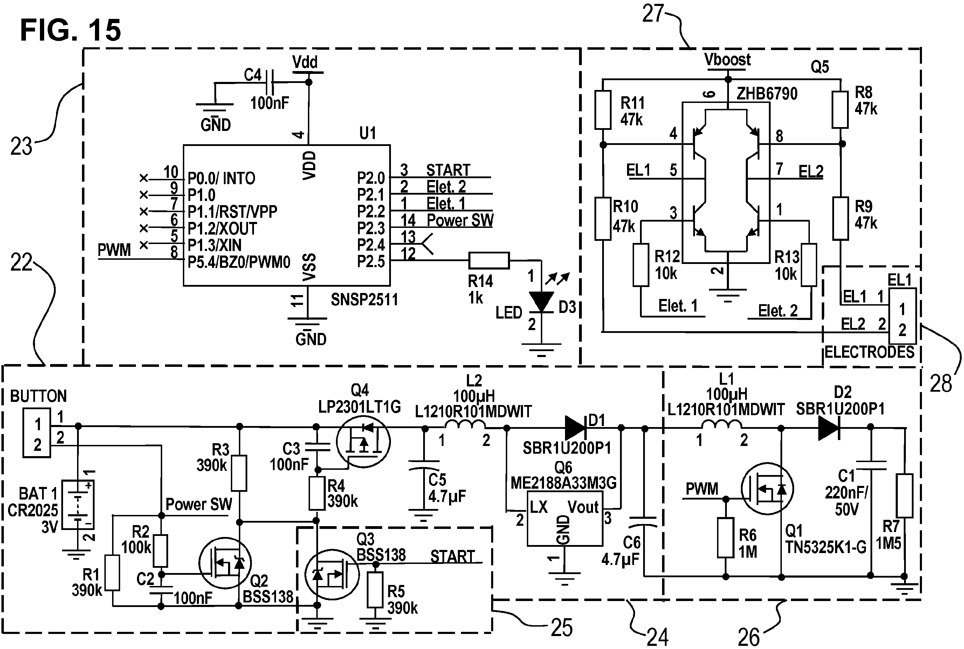

The block diagram of Figure 14 illustrates the internal components of the electronic module of this innovative equipment, namely: Power source module (15), step-up regulator module (16), micro controller module (17), power supply seal module (18), boost source module (19), H-bridge module (20), and electrode output module (21). These components will be explained in details below, together with the description of this innovative electrical equipment, shown in attached Figure 15.

As verified in said Figure 15, the circuit (22), which refers to the Power Supply Module (15), supplies all the other circuits with the voltage supplied by the battery. When MOSFET "Q2" is saturated with the signal coming from the power bottom, it will saturate MOSFET "Q4", energizing the circuit (24), which refers to the Step-up Regulator Module (16).

Circuit (24) has a "Q6" Step-Up Regulator Module, which acquires the voltage of the battery and raises it to 3.3 V. This output voltage is filtered by the capacitor "C6", and then supplied to the circuit (26) and to power the microcontroller.

In the circuit (23), which refers to the Microcontroller Module (17), there is microcontroller "U1", which is responsible for controlling all functions of the equipment. Once it has been powered, the "D3" LED flashes in an intermittent manner to indicate the operation of the equipment. Furthermore, the microcontroller then monitors the power button signal, sends the PWM signal to "Q1", sends the signals to control the H-Bridge and also sends a signal to "Q3" in the circuit (25), which refers to the Power Seal Module (18), responsible for keeping the MOSFET "Q4" of the circuit (22) saturated, which makes it unnecessary for the Power button to be pressed to allow the equipment to remain energized.

Circuit (26) which refers to Boost Source Module (19) is a boost-type DC/DC converter whose function is to raise the 3.3V voltage range to the level that will be used in electrostimulation. The converter is controlled by a PWM signal applied to the MOSFET "Q1", and the output voltage is sent to the circuit (27) through the capacitor "C1".

Circuit (27), which refers to a H-Bridge Module (20), has the function of discharging in the electrodes the energy stored in the capacitor "C1" of the boost source. Another feature is to reverse the polarity of current flowing through these electrodes, said inversion occurring according to the digital signals emitted by the microcontroller. The width of the pulses outcoming from the H-bridge is also controlled by the same signal which controls polarity reversal, and then, finally, these stimuli are conducted to the user through the electrodes represented by the block (28) in Figure 15, which refers to the Electrode Output Module (21) in the block diagram of Figure 14.

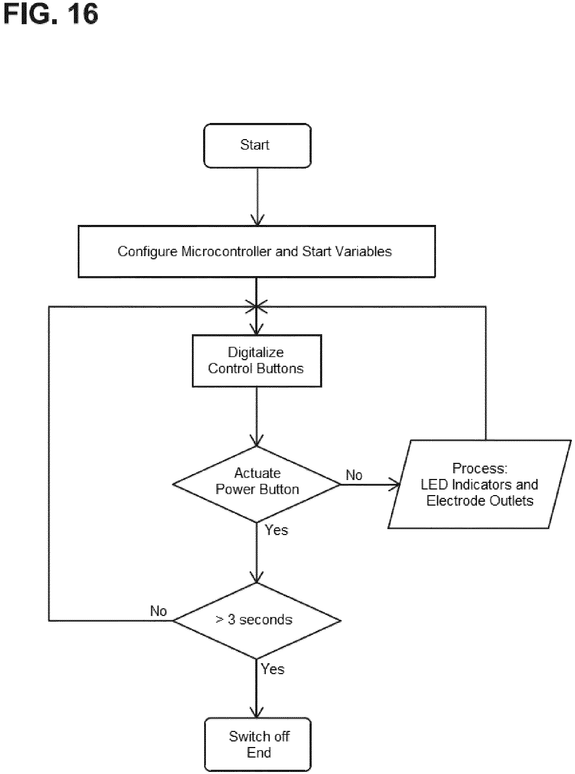

Figure 16 is a flowchart of the software specifically developed for this innovative electrostimulation protocol for this innovative headache control electrostimulation protocol.

As previously mentioned, not only does the new protocol of electrostimulation created by the Inventor(s) effectively reduce nerve fiber accommodation, but it makes also possible to reduce current consumption in the operations of the electrostimulation equipment, allowing the use of a disposable coin-shaped lithium-ion battery (5), model CR20XX, with smaller sizes, low cost and lower charge capacity, but sufficient to meet the current lower current consumption thanks to the random intensity variations determined by the innovative electric stimulation protocol.

Hence, it has become possible to develop the innovative electric stimulation equipment, whose electrostimulation set (1) is small, cheap, disposable and portable, making it possible for users to use it at any time and in any place, at home or at work.

Furthermore, thanks to the arrangement of the novel support (2), which also integrates this innovative equipment, the performed electrostimulation sessions have been carried out in a safer and more efficient manner, and in various regions of the user's face, more particularly between the supraorbital and temporal regions of his/her head, providing even more comfort to the user.

## Claims

1. A PROCESS FOR ESTABLISHING AN ELECTROSTIMULATION PROTOCOL FOR HEADACHE CONTROL **characterized in that** it provides a random variation of electrical pulse intensity, respecting the stimulation efficacy limits, wherein said random variation is comprised in the range of 10 V, and said electrical pulses exhibit a square wave shape, are monopolar, have a width (or duration) between 60 µs and 100 µs, and a frequency between 40 Hz and 70 Hz; and said electrostimulation protocol further involves the following steps:
a) intensity ramp from 0V to 25V peak (with 500Ω charge), for 2 seconds;
b) 1^{st} continuous mode period, maintained for 5 minutes;
c) 1^{st} intermittent mode period, maintained for 2 minutes;
d) 2^{nd} continuous mode period, maintained for 5 minutes;
e) 2^{nd} intermittent mode period, maintained for 2 minutes;
f) 3r^{d} continuous mode period, maintained for 5 minutes;
g) 3^{rd} intermittent mode period, maintained for 2 minutes;
h) end of the protocol, after 21 minutes and 2 seconds.

2. The PROCESS FOR ESTABLISHING AN ELECTROSTIMULATION PROTOCOL FOR HEADACHE CONTROL, according to claim 1, **characterized in that** electrical pulse intensity varies randomly, preferably within the range of 5V.

3. The PROCESS FOR ESTABLISHING AN ELECTROSTIMULATION PROTOCOL FOR HEADACHE CONTROL, according to claim 1, **characterized in that**
the width (or duration) of the electrical pulses is 80 µs, and/or that
the frequency of the electrical pulses is preferably 55 Hz.

4. The PROCESS FOR ESTABLISHING AN ELECTROSTIMULATION PROTOCOL FOR HEADACHE CONTROL, according to claim 1, **characterized in that** it provides two electrostimulation modes, namely, continuous mode and intermittent mode.

5. The PROCESS FOR ESTABLISHING AN ELECTROSTIMULATION PROTOCOL FOR HEADACHE CONTROL, according to claims 1 and 4, **characterized in that** pulse bursts are continuously applied, in a continuous mode, with duration from 500 milliseconds to 2 seconds, and with sequentially inverted polarity.

6. PROCESS FOR ESTABLISHING AN ELECTROSTIMULATION PROTOCOL FOR HEADACHE CONTROL, according to claim 5, **characterized in that** said pulse bursts exhibit a duration of 1 second.

7. The PROCESS FOR ESTABLISHING AN ELECTROSTIMULATION PROTOCOL FOR HEADACHE CONTROL, according to claims 1 and 4, **characterized in that**, in the intermittent mode, pulse bursts lasting 1-4 seconds and with sequentially inverted polarity, with a time intervals between the pulse bursts, are applied in an intermittent fashion, wherein said interval lasts 1-4 seconds.

8. The PROCESS FOR ESTABLISHING AN ELECTROSTIMULATION PROTOCOL FOR HEADACH CONTROL, according to claim 7, **characterized in that** said pulse bursts exhibit a duration of 3 second.

9. The PROCESS FOR ESTABLISHING AN ELECTROSTIMULATION PROTOCOL FOR HEADACHE CONTROL, according to claim 7, **characterized in that** said time interval between the pulse bursts exhibits a duration of 3 second.

10. The PROCESS FOR ESTABLISHING AN ELECTROSTIMULATION PROTOCOL FOR HEADACHE CONTROL, according to claim 1, **characterized in that** it optionally discloses the simultaneous use of other strategies both to avoid nerve fiber accommodation, used together with the random variation of pulse intensity.

11. The PROCESS FOR ESTABLISHING AN ELECTROSTIMULATION PROTOCOL FOR HEADACHE CONTROL, according to claims 1 and 10, **characterized in that**
one of said strategy is the use of monopolar pulse bursts, that
the other of said strategies is pulse burst polarity inversion, and/or that
one of said strategy is the addition of intermittent stimulation modes.

12. A PORTABLE ELECTROSTIMULATION EQUIPMENT FOR HEADACHE CONTROL USING SAID PROTOCOL, wherein said equipment comprises two parts, an electrostimulation set (1) itself, and a support (2), in which said electrostimulation set (1) is installed, **characterized in that** said electrostimulation set (1) comprises a cylindrical compartment (3) formed by a front body (3a), and a back cover (3b) which are engageable with one another and which houses an electrical circuit board (4) and a coin-shaped ion-lithium ion battery (5), model CR20XX; wherein, on the outer back face of the cover (3b) there are recesses (6a) housing the respective electrodes (6), covered by the respective gel layers protected by removable protective sheets (7); The body (3a) is endowed with a central hole (8), from which a front small tube (9) projects outwards, whose free end extends outwardly on an external flange (10), said small tube (9) being pierced by a pin (11) constituting the power button of the equipment, electrically interconnected to the electrical circuit board (4), said small tube (9) and flange (10) constituting the means for engaging the electrostimulation set (1) on the support ( 2); said support (2) is configured by a lying U-shaped rod (12), the arched central portion (12a) of which coincides with the user's forehead, and whose lateral portions (12b), flanking his/her temples, extend backwards in end portions (12c) resting on his/her ears; Duly fitted to the free ends of said end portions (12c) of the rod (12), there are stylized C-shaped adjustment pieces 13; said rod (12) having a medial longitudinal groove, wherein said groove (14) configures the rail with which the electrostimulation set (1) is engaged, and along which said set (1) is moved; the following internal components are further provided: power source module (15), step-up regulator module (16), micro controller module (17), power supply seal module (18), boost source module (19), H-bridge module (20), and electrode output module (21).

13. The PORTABLE ELECTROSTIMULATION EQUIPMENT FOR HEADACHE CONTROL USING SAID PROTOCOL, according to claim 12, **characterized in that** said disposable battery (5) is a 3V one.

14. The PORTABLE ELECTROSTIMULATION EQUIPMENT FOR HEADACHE CONTROL USING SAID PROTOCOL, according to claim 12, **characterized in that** it comprises the electrical scheme shown in Figure 15.

15. The PORFranTABLE ELECTROSTIMULATION EQUIPMENT FOR HEADACHE CONTROL USING SAID PROTOCOL, according to claim 12, **characterized in that** it comprises the flowchart shown in Figure 16.
